# EUROPEAN PATENT APPLICATION

(11) **EP 2 476 410 A1**
(43) Date of publication of application: **18.07.2012**
(21) Application number: 11305033.0
(22) Date of filing: 13.01.2011
(51) Int. Cl.: A61K 9/00, A61K 38/00

(54) **An analgesic peptide-containing composition for transbuccal administration**

(71) Applicant: Theralpha, 06560 Valbonne (FR)
(72) Inventor: Lazdunski, Michel, 06000, Nice (FR)
(74) Representative: Icosa

(57) **Abstract**

The present invention relates to a pharmaceutical composition comprising a peptide of 5 to 50 amino acids which comprises the amino acid sequence NPFPTX₁X₂KRX₃X₄ (SEQ ID NO: 2) wherein X₁, X₂, X₃, and X₄ may be the same or different and each is an amino acid residue, wherein said composition is in a form suitable for a transbuccal administration and use thereof for preventing or treating pain.

SEQUENCE LISTING

<110>  THERALPHA

<120>  Analgesic composition for transbuccal administration

<130>  CV - 122/EP

<160>  7

<170>  PatentIn version 3.5

<210>  1
<211>  5
<212>  PRT
<213>  artificial

<220>
<223>  motif NPFPT

<400>  1

Asn Pro Phe Pro Thr
1               5


<210>  2
<211>  11
<212>  PRT
<213>  artificial

<220>
<223>  analgesic peptide


<220>
<221>  MISC_FEATURE
<222>  (6)..(6)
<223>  X is absent or W

<220>
<221>  MISC_FEATURE
<222>  (7)..(7)
<223>  X is absent or R

<220>
<221>  MISC_FEATURE
<222>  (10)..(10)
<223>  X is P or K

<220>
<221>  MISC_FEATURE
<222>  (11)..(11)
<223>  X is absent or G or H

<400>  2

Asn Pro Phe Pro Thr Xaa Xaa Lys Arg Xaa Xaa
1               5                   10


<210>  3
<211>  11
<212>  PRT
<213>  artificial

<220>
<223>  prohanin

<400>  3

Asn Pro Phe Pro Thr Trp Arg Lys Arg Pro Gly
1               5                   10


<210>  4
<211>  9
<212>  PRT
<213>  artificial

<220>
<223>  analgesic peptide

<400>  4

Asn Pro Phe Pro Thr Arg Lys Arg Pro
1               5


<210>  5
<211>  10
<212>  PRT
<213>  artificial

<220>
<223>  analgesic peptide

<400>  5

Asn Pro Phe Pro Thr Trp Arg Lys Arg Pro
1               5                   10


<210>  6
<211>  10
<212>  PRT
<213>  artificial

<220>
<223>  analgesic peptide

<400>  6

Asn Pro Phe Pro Thr Trp Lys Arg Lys His
1               5                   10


<210>  7
<211>  4
<212>  PRT
<213>  artificial

<220>
<223>  motif NPFP

<400>  7

Asn Pro Phe Pro
1

## Description

### FIELD OF THE INVENTION

The present invention relates to a composition comprising an analgesic peptide for use in the prevention or treatment of pain, wherein the composition is administrated via a transbuccal route.

### BACKGROUND OF THE INVENTION

The analgesic prohanin, described in US6613745, is a peptide of 5 to 50 amino acids comprising the sequence NPFPT (SEQ ID NO: 1) used in a method for inducing or facilitating analgesia in a subject in need thereof. US6613745 mainly described an intravenous or intraperitoneal injection or an anal administration of the peptide for inducing analgesia.

It is widely recognized that administration of drugs by injection can be both inconvenient and unpleasant for the patient, particularly when the administration has to be repeated at regular intervals for long periods.

In addition, adequate management of pain, especially acute pain, remains a serious medical problem. Acute pain often arises quickly, and lasts a relatively short time, as compared to long-term chronic pain. This type of pain generally comes on suddenly, after a trauma or a surgery for example, and may be further accompanied by anxiety, emotional distress and/or depression.

Thus, there is a need for administrating medicament by more acceptable non-invasive alternative routes that provide the desired fast convenient relief from pain.

However, a considerable and well known problem with the administration of peptides is that they are susceptible to rapid acid and enzyme-induced degradation when administered orally. The transbuccal route has been accepted as a potential non-invasive route of drug administration, with advantages of avoidance of the first-pass metabolism, sustained therapeutic action and better patient compliance.

The major challenges of transbuccal administration of drugs are limited absorption area and the barrier properties of the buccal mucosa, which implies low drug bioavailability. It is known in the art that rapid uptake and sustained delivery of lipophilic drugs can be achieved; however buccal delivery of high-molecular-mass drugs such as peptides often results in low bioavailability (less than 1%). Conventionally, the buccal route cannot be used for the delivery of macromolecular drugs such as peptides owing to limited transport across the epithelial membrane. For example, it is known in the art that the bioavailability of oxytocin (9 amino acids peptide, 1007.19 Da molecular weight) by the sublingual route is very low (0.007-0.07%) (De Groot et al. J. Pharm. Pharmacol. 1995, 47(7): 571-5). Another example is the bioavailability of about 0.25% of the peptide desmopressin (9 amino acids peptide, 1183 Da molecular weight) by the sublingual route.

The Applicant made the surprising observation that the prohanin, although being a peptide of approximately 1000 Da of molecular weight, shows a bioavailability of more than 40 % when administered via a sublingual route.

The present invention thus relates to a pharmaceutical composition comprising a peptide of 5 to 50 amino acids having the sequence NPFPT (SEQ ID NO: 1), being in a form suitable for a transbuccal administration and the use thereof via a transbuccal administration for inducing analgesia and preventing or treating pain.

### SUMMARY

One object of the invention is a pharmaceutical composition comprising a peptide of 5 to 50 amino acids which comprises the amino acid sequence NPFPTX₁X₂KRX₃X₄ (SEQ ID NO: 2) wherein X₁, X₂, X₃, and X₄ may be the same or different and each is an amino acid residue, wherein said composition is in a form suitable for a transbuccal administration.

In one embodiment of the invention, the peptide comprises the acid sequence NPFPTX₁X₂KRX₃X₄ (SEQ ID NO: 2) wherein X₁ is absent or is W, X₂ is absent or is R, X₃ is P or K and X₄ is absent or is G or H.

In another embodiment of the invention, the peptide comprises the amino acid sequence NPFPTWRKRPG (SEQ ID NO: 3), NPFPTRKRP (SEQ ID NO: 4), NPFPTWRKRP (SEQ ID NO: 5), NPFPTWKRKH (SEQ ID NO: 6) or a derivative, homologue, analogue or mimetic of SEQ ID NO: 3-6.

In another embodiment of the invention, the composition is in a form suitable for transbuccal administration selected among liquid solutions, orally disintegrating tablets, bioadhesive tablets, microspheres or nanospheres of any shape, film, lollipop, chewing-gum, mouthwash, toothpaste, buccal drops and spray.

In another embodiment of the invention, the composition is in a form of a spray. In another embodiment of the invention, the composition is in a form of an orally disintegrating tablet.

Another object of the invention is the pharmaceutical composition as described here above, for use in inducing or facilitating analgesia in a subject in need thereof, wherein said peptide or pharmaceutical composition is administered via a transbuccal route. Another object of the invention is the pharmaceutical composition as described here above, for use in preventing or treating pain in a subject in need thereof, wherein said peptide or pharmaceutical composition is administered via a transbuccal route.

In one embodiment of the invention, the pharmaceutical composition is administered via a sublingual route.

In another embodiment of the invention, the pain is an inflammatory pain or an acute or chronic pain. In another embodiment of the invention, the pain is a symptom of a condition associated with nitric oxide synthase.

### DETAILED DESCRIPTION OF THE INVENTION

### Definitions

As used herein, the term "oral cavity" refers to the mouth or mouth region or the surface on which the mouth is located. It includes the floor of the sublingual (mouth), the palate, the inside of the jaws, the interior of the lips, the gums and the interior of the jaws.

As used herein, the term "transbuccal administration" refers to the delivery of the peptide across any tissue of the mouth, particularly the sublingual, buccal, gingival, and palatal mucosal tissues.

As used herein, the term "bioadhesion" means an adhesion to biological surfaces, including mucous membranes which are covered by mucus and mucous membranes which are not covered by mucus. The term "mucoadhesion" can be used interchangeably with "bioadhesion".

As used herein, the terms "slow release" or "sustained release" can be used interchangeably and mean that the peptide is released immediately and then over a prolonged period of time.

The term "analgesia" is used herein in its broadest sense to include any form of pain relief ranging from complete removal of pain to a reduced level of pain. The pain relief may be considered a reduced sensibility to pain without loss of consciousness. The pain relief may be localized or systemic. Pain includes acute pain, sharp localized pain, more centralized pain and any other form of sensation which contributes to discomfort. An "analgesic" is considered herein to be a molecule which induces analgesia.

As used herein, "pain" encompasses a wide range of clinical manifestations, and it has a broad meaning. Pain perception is highly subjective, and different people experience pain in different ways and with greatly different intensities. The International Association for the Study of Pain defines pain as "an unpleasant sensory and emotional experience associated with actual or potential tissue damage, or described in terms of such damage." More simply stated, pain includes any sensory experience that causes suffering and is associated with an unpleasant awareness of one's own body. Nonlimiting types and causes of pain include neuralgia, myalgia, hyperalgesia, hyperpathia, neuritis, and neuropathy. Pain is often a symptom of an underlying physiological abnormality, such as cancer or arthritis. Some types of pain have no clearly identified causes, such as migraine headache pain. Pain may also be caused by physical trauma, such as burns or surgery. Viral infections, such as Herpes zoster (chicken pox and shingles), can also cause pain. Withdrawal from chemical dependence on alcohol or drugs of abuse is also often associated with pain symptoms. Accordingly, "pain" is understood herein to have a very broad meaning and its claimed uses should not be construed as being limited to any particular malady or condition.

As used herein, the term "peptide" encompasses a proteinaceous molecule comprising from about 2 amino acid residues to about 50 amino acid residues. Preferably, the peptide comprises from about 3 amino acid residues to about 40 amino acid residues. Even more preferably, the peptide comprises from about 4 amino acid residues to about 30 amino acid residues. Particularly preferred embodiments include peptides comprising from about 4 amino acid residues to about 20 amino acid residues such as from 4 to 15 and 4 to 10 amino acid residues.

As used herein, the term "treating" includes prophylaxis of the specific disorder or condition, or alleviation of the symptoms associated with a specific disorder or condition and/or preventing or eliminating said symptoms. A "prophylactic" treatment is a treatment administered to a subject who does not exhibit signs of a disease or exhibits only early signs of the disease for the purpose of decreasing the risk of developing pathology associated with the disease. A "therapeutic" treatment is a treatment administered to a subject who exhibits signs of pathology for the purpose of diminishing or eliminating those signs. A "therapeutically effective amount" of a compound is that amount of compound which is sufficient to provide a beneficial effect to the subject to whom the compound is administered. As used herein, "preventing or treating pain" includes: (i) preventing the pain, i.e. causing the clinical symptoms of the pain not to develop in a subject that may be exposed to or predisposed to the pain but does not yet experience or display symptoms of the pain, (ii) inhibiting and managing the pain, i.e. arresting or reducing the development of pain or its cause(s), or (iii) relieving the pain, i.e. causing regression or cessation of the pain or its cause(s).

As used herein "pharmaceutically acceptable vehicles" include carriers, excipients and stabilizers. The formulations can be prepared as set forth in Remington's Pharmaceutical Sciences 16th edition, Osol A. editor (1980). Examples of carriers, excipients and carriers include saline, PBS, buffers such as phosphate, citrate and other organic acids; antioxidants such as ascorbic acid, low molecular weight polypeptides; proteins such as serum albumin, immunoglobulins, gelatins; hydrophilic polymers such as PVP; amino acids of glycine, glutamine, arginine, lysine or asparagines; carbohydrates including glucose, mannose or dextrins; sugar alcohols including mannitol or sorbitol; salt-forming counterions such as sodium and/or nonionic surfactants such as Tween®.

As used herein "consisting essentially of" means that the pharmaceutical composition can contain other minor ingredients that do not affect the physiological action of the first and/or second active ingredients of the pharmaceutical composition described herein.

### The invention

The Applicants made the surprising observation that administration of the prohanin via a sublingual route showed a far better bioavailability (44%) than an oral administration (4%) or intraperitoneal administration (0.5%). In addition, they observed that the plasma concentration of the peptide was still superior to 100 ng/ml 1 hour after administration compared to approximately undetectable via the other routes of administration.

Contrary to the knowledge of prior art on transbuccal bioavailability of peptides, the prohanin can thus be efficiently administered via a transbuccal route. This is of great interest as a lower dosage of the pharmaceutical composition can be used for inducing or facilitating analgesia. A lower dosage is of particular interest in the present invention to avoid tolerance to the pharmaceutical composition.

One object of the present invention is a pharmaceutical composition comprising or consisting essentially of a peptide which comprises the amino acid sequence NPFPT (SEQ ID NO: 1), wherein said composition is in a form suitable for a transbuccal administration.

According to the invention, the peptide is 5 to 50 amino acids length, preferably from 5 to 40 amino acids length, from 5 to 30 amino acids length, from 5 to 25 amino acids length, more preferably from 5 to 24, 23, 22, 21, 20, 19, 18, 17, 16, 15, 14, 13, 12, 11, 10, 9, 8, 7, 6 amino acids length.

According to the invention, said peptide may be the peptide as described in US6613745, which is incorporated herein by reference.

According to the invention, said peptide is a fragment or portion of the α-neurotoxin from *O*. *Hannah* snake venom capable of inducing or facilitating analgesia (References Swiss Prot: Q53B58.1).

According to the invention, said peptide is in an isolated or purified form. The peptide may be produced by chemical synthetic techniques or may be produced by recombinant DNA technology as well known in the art.

According to the invention, the peptide may be glycosylated or unglycosylated and/or may contain a range of other molecules fused, linked, bound or otherwise associated to the peptide such as amino acids, lipids, carbohydrates or other peptides, polypeptides or proteins.

In one embodiment of the invention, said peptide comprises the amino acid sequence NPFPTX₁X₂KRX₃X₄ (SEQ ID NO: 2), wherein X₁, X₂, X₃, and X₄ may be the same or different and each is an amino acid residue.

Preferably, X₁ is absent or is W, X₂ is absent or is R, X₃ is P or K and X₄ is absent or is G or H.

In another embodiment of the invention, the peptide comprises the amino acid sequence NPFPTWRKRPG (SEQ ID NO: 3).

In another embodiment of the invention, the peptide comprises the amino acid sequence NPFPTRKRP (SEQ ID NO: 4).

In another embodiment of the invention, the peptide comprises the amino acid sequence NPFPTWRKRP (SEQ ID NO: 5).

In another embodiment of the invention, the peptide comprises the amino acid sequence NPFPTWKRKH (SEQ ID NO: 6).

In one embodiment of the invention, the peptide may comprise derivatives, homologues, analogues or mimetics of SEQ ID NO: 1, 2, 3, 4, 5 or 6.

Reference herein to "derivatives" includes parts, fragments and portions of the subject peptides. A derivative also includes a single or multiple amino acid substitution, deletion and/or addition. Homologues include functionally, structurally or stereochemically similar peptides from venom from the same species of snake or from within the same genus or family of snake. For example, a homologue would include a peptide having, for example, the amino acid sequence NPFP (SEQ ID NO:7) from another α-neurotoxin from another member of the Elapidae family. Alternatively, the amino acids NPFP (SEQ ID NO:7) may be replaced by amino acids having a similar charge, stereochemistry, conformation or peptide conformation influencing ability. All such homologues are contemplated by the present invention.

Analogues and mimetics include molecules which contain non-naturally occurring amino acids or which do not contain amino acids but nevertheless behave functionally the same as the peptide. Natural product screening is one useful strategy for identifying analogues and mimetics.

However, analogues of the subject peptides contemplated herein include modifications to side chains, incorporation of unnatural amino acids and/or their derivatives during peptide synthesis and the use of crosslinkers and other methods which impose conformational constraints on the peptide molecule or their analogues. Examples of side chain modifications contemplated by the present invention include modifications of amino groups such as by reductive alkylation by reaction with an aldehyde followed by reduction with NaBH4; amidination with methylacetimidate; acylation with acetic anhydride; carbamoylation of amino groups with cyanate; trinitrobenzylation of amino groups with 2, 4, 6-trinitrobenzene sulphonic acid (TNBS); acylation of amino groups with succinic anhydride and tetrahydrophthalic anhydride; and pyridoxylation of lysine with pyridoxal-5-phosphate followed by reduction with NaBH4.

The guanidine group of arginine residues may be modified by the formation of heterocyclic condensation products with reagents such as 2,3-butanedione, phenylglyoxal and glyoxal.

The carboxyl group may be modified by carbodiimide activation via O-acylisourea formation followed by subsequent derivitisation, for example, to a corresponding amide. Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ring of a histidine residue may be accomplished by alkylation with iodoacetic acid derivatives or N-carbethoxylation with diethylpyrocarbonate.

Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, omithine, sarcosine, 4-amino-3-hydroxy-6-methylheptanoic acid, 2-thienyl alanine and/or D-isomers of amino acids. A list of unnatural amino acid contemplated herein is shown in Table 1.

**Table 1**

| **Non-conventional amino acid** | **Code** | **Non-conventional amino acid** | **Code** |
|---|---|---|---|
| [alpha]-aminobutyric acid | Abu | L-N-methylalanine | Nmala |
| [alpha]-amino-a-methylbutyrate | Mgabu | L-N-methylarginine | Nmarg |
| aminocyclopropane-carboxylate | Cpro | L-N-methylasparagine | Nmasn |
| L-N-methylaspartic acid | Nmasp | L-N-methylglutamine | Nmgln |
| aminoisobutyric acid | Aib | L-N-methylcysteine | Nmcys |
| aminonorbomyl-carboxylate | Norb | L -N-methylglutamic acid | Nmglu |
| cyclohexylalanine | Chexa | L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | [alpha]-methyl-aminoisobutyrate | Maib |
| D-valine | Dval | [alpha]-methyl-[gamma]-aminobutyrate | Mgabu |
| D-[alpha]-methylalanine | Dmala | [alpha]-methylcyclohexylalanine | Mchexa |
| D-[alpha]-methylarginine | Dmarg | [alpha] -methylcylcopentylalanine | Mcpen |
| D-[alpha]-methylasparagine | Dmasn | [alpha]-methyl-[alpha]-napthylalanine | Manap |
| D-[alpha] -methylaspartate | Dmasp | [alpha]-methylpenicillamine | Mpen |
| D-[alpha]-methylcysteine | Dmcys | N-(4-aminobutyl)glycine | Nglu |
| D-[alpha]-methylglutamine | Dmgln | N-(2-aminoethyl)glycine | Naeg |
| D-[alpha]-methylhistidine | Dmhis | N-(3-aminopropyl)glycine | Norn |
| D-[alpha]-methylisoleucine | Dmile | N-amino-[alpha]-methylbutyrate | Nmaabu |
| D-[alpha]-methylleucine | Dmleu | [alpha]-napthylalanine | Anap |
| D-[alpha]-methyllysine | Dmlys | N-benzylglycine | Nphe |
| D-[alpha]-methylmethionine | Dmmet | N-(2-carbamylethyl)glycine | Ngln |
| D-[alpha]-methylornithine | Dmorn | N-(carbamylmethyl)glycine | Nasn |
| D-[alpha]-methylphenylalanine | Dmphe | N-(2-carboxyethyl)glycine | Nglu |
| D-[alpha]-methylproline | Dmpro | N-(carboxymethyl)glycine | Nasp |
| D-[alpha]-methylserine | Dmser | N-cyclobutylglycine | Ncbut |
| D-[alpha]-methylthreonine | Dmthr | N-cycloheptylglycine | Nchep |
| D-[alpha]-methyltryptophan | Dmtrp | N-cyclohexylglycine | Nchex |
| D-[alpha]-methyltyrosine | Dmty | N-cyclodecylglycine | Ncdec |
| D-[alpha]-methylvaline | Dmval | N-cylcododecylglycine | Ncdod |
| D-N-methylalanine | Dnmala | N-cyclooctylglycine | Ncoct |
| D-N-methylarginine | Dnmarg | N-cyclopropylglycine | Ncpro |
| D-N-methylasparagine | Dnmasn | N-cycloundecylglycine | Ncund |
| D-N-methylaspartate | Dnmasp | N-(2,2-diphenylethyl)glycine | Nbhm |
| D-N-methylcysteine | Dnmcys | N-(3,3-diphenylpropyl)glycine | Nbhe |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl))glycine | Nser |
| D-N-methylisoleucine | Dnmile | N-(imidazolylethyl))glycine | Nhis |
| D-N-methylleucine | Dnmleu | N-(3-indolylyethyl)glycine | Nhtrp |
| D-N-methyllysine | Dnmlys | N-methyl-[gamma]-aminobutyrate | Nmgabu |
| N-methylcyclohexylalanine | Nmchexa | D-N-methylmethionine | Dnmmet |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl)glycine | Nval |
| D-N-methyltyrosine | Dnmtyr | N-methyla-napthylalanine | Nmanap |
| D-N-methylvaline | Dnmval | N-methylpenicillamine | Nmpen |
| [gamma]-aminobutyric acid | Gabu | N-(p-hydroxyphenyl)glycine | Nhtyr |
| L-t-butylglycine | Tbug | N-(thiomethyl)glycine | Ncys |
| L-ethylglycine | Etg | penicillamine | Pen |
| L-homophenylalanine | Hphe | L-[alpha]-methylalanine | Mala |
| L-[alpha]-methylarginine | Marg | L-[alpha]-methylasparagine | Masn |
| L-[alpha]-methylaspartate | Masp | L-[alpha] -methyl-t-butylglycine | Mtbug |
| L-[alpha]-methylcysteine | Mcys | L-methylethylglycine | Metg |
| L-[alpha]-methylglutamine | Mgln | L-[alpha] -methylglutamate | Mglu |
| L-[alpha]-methylhistidine | Mhis | L-[alpha]-methylhomophenylalanine | Mhphe |
| L-[alpha]-methylisoleucine | Mile | N-(2-methylthioethyl)glycine | Nmet |
| L-[alpha]-methylleucine | Mleu | L-[alpha]-methyllysine | Mlys |
| L-[alpha]-methylmethionine | Mmet | L-[alpha]-methylnorleucine | Mnle |
| L-[alpha]-methylnorvaline | Mnva | L-[alpha]-methylornithine | Morn |
| L-[alpha]-methylphenylalanine | Mphe | L-[alpha]-methylproline | Mpro |
| L-[alpha]-methylserine | Mser | L-[alpha]-methylthreonine | Mthr |
| L-[alpha]-methyltryptophan | Mtrp | L-[alpha]-methyltyrosine | Mtyr |
| L-[alpha] -methylvaline | Mval | L-N-methylhomophenylalanine | Nmhphe |
| N-(N-(2,2-diphenylethyl) carbamylmethyl)glycine | Nnbhm | N-(N-(3,3-diphenylpropyl) | Nnbhe |
| carbamylmethyl)glycine 1-carboxy-1-(2,2-diphenyl-ethylamino)cyclopropane | Nmbc | | |

Also included are peptides that contain, consist essentially of, or consist of an amino acid sequence that differs from the sequence of SEQ ID NO:2-6 by one, two, three, four, five, six, seven, eight, nine, ten, eleven, twelve, thirteen, or fourteen amino acid substitutions, additions, or deletions. They can be at any position, e.g., internal or terminal (e.g., at the amino or carboxy terminus).

Crosslinkers can be used, for example, to stabilize 3D conformations, using homo-bifunctional crosslinkers such as the bifunctional imido esters having (CH2)n spacer groups with n=1 to n=6, glutaraldehyde, N-hydroxysuccinimide esters and heterobifunctional reagents which usually contain an amino-reactive moiety such as N-hydroxysuccinimide and another group specific-reactive moiety such as maleimido or dithio moiety (SH) or carbodiimide (COOH). In addition, peptides can be conformationally constrained by, for example, incorporation of C[alpha] and N[alpha]-methylamino acids, introduction of double bonds between C[alpha] and C[beta] atoms of amino acids and the formation of cyclic peptides or analogues by introducing covalent bonds such as forming an amide bond between the N and C termini, between two side chains or between a side chain and the N or C terminus.

In one embodiment of the invention, the form of the pharmaceutical composition suitable for a transbuccal administration is selected among liquid or aqueous solutions, orally disintegrating tablets; bioadhesive tablets, microspheres or nanospheres that can be formulated in any shape such as rectangular, circular, square, oval and the like; film; lollipop; chewing-gum; mouthwash; toothpaste; emulsions; buccal drops and buccal spray.

In one embodiment of the invention, the pharmaceutical composition may comprise pharmaceutically acceptable excipients as well as optional flavoring agents, pressurized propellants, preservatives, emulsifiers, buffers, colorants and the like.

Examples of excipients include viscosity modulating materials (e.g. polymers, sugars, sugar alcohols, gums, clays, silicas, and the like (e.g., polyvinylpyrrolidone (PVP)) (from about 0.01% to about 65% by weight); preservatives (e.g., ethanol, benzyl alcohol, propylparaben and methylparaben) (from about 0.001% to about 10% by weight); flavoring agents, sweeteners (e.g., sugars (sucrose, glucose, dextrose, maltose, fructose, etc.), artificial sweeteners (saccharin, aspartame, acesulfame, sucralose), or sugar alcohols (mannitol, xylitol, lactitol, maltitol syrup) (from about 0.001% to about 65% by weight). Still other examples of excipients include buffers and pH-adjusting agent (e.g., sodium hydroxide, citrate, and citric acid) (from about 0.01% to about 5% by weight); coloring agents (from about 0.001% to about 5% by weight); fragrances (from about 0.001% to about 1% by weight); chelating agents (e.g., EDTA) (from about 0.001% to about 1% by weight); UV absorbers (from about 0.001% to about 10% by weight), and antifoam agents (e.g., low molecular weight alcohols, dimethicone, simethicone) (from about 0.001% to about 5% by weight).

The pharmaceutical composition of the invention may be in a liquid form (homogenous suspension, solution or combination thereof) and thus comprises one or more pharmaceutically acceptable liquids (from about 30% to about 99.9% by weight). Examples of pharmaceutically acceptable liquids include, but are not restricted to, water, ethanol, dimethyl sulfoxide, propylene glycol, polyethylene glycol, propylene carbonate, pharmaceutically acceptable oils and the like.

In another embodiment of the invention, the pharmaceutical composition may further comprise a bioadhesion or mucoadhesion promoting compound in order to limit the variability of administrated doses or a penetration enhancer, in order to enhance the peptide absorption.

Examples of bioadhesion or mucoadhesion promoting compound include, but are not limited to, sodium EDTA, Menthol, polysorbate 80, phosphatidylcholine, sodium glycholate, sodium glycodeoxycholate, sodium lauryl sulfate, sodium salicylate, chitosan, lethylpyrrolidinone chitosan, sodium taurocholate, benzalkonium chloride, azone®, sodium taurodeoxycholate, cetylpyridinium chloride, lauric acid, polyethylene glycol, cyclodextrin, laureth-9, lysalbinic acid.

In one embodiment of the invention, a spray formulation of the pharmaceutical composition as described here above can be provided in metered dosages so that a predetermined amount of the peptide is properly administered to the subject in a pharmaceutically effective amount. For example, the spray formulations may be packaged as a bulk liquid containing multiple doses in a pump spray system comprising a sealed container fitted with a metering pump. Typically, a subject is treated by sublingual self-administration, such as by one or more actuations from a spray pump. An advantage of spray delivery examples herein is the ability to titrate subjects by single doses as required through single, discrete actuations. Additional advantages of sublingual spray formulations include their ease of use, especially when self-administered in the absence of an attending health care professional.

Pump action sprays are characterized in requiring the application of external pressure for actuation, for example, external manual, mechanical or electrically initiated pressure. This is in contrast to pressurized systems, e.g., propellant-driven aerosol or compressed gas sprays, where actuation is typically achieved by controlled release of pressure e.g., by controlled opening of a valve. In certain embodiments, pump sprays are preferred as the use of a pump spray with the formulations herein allows for the administration of droplets or particles having a small mean diameter and a controllable size distribution of droplets. In other embodiments, pressurized systems containing a reservoir of pressurized propellant gas (e.g., carbon dioxide, nitrogen, chlorofluorocarbons, hydrofluoroalkanes, etc.) may produce suitable particles or droplets. Liquid droplets or particles having a diameter that is too small have the potential to enter into the lungs of a human upon administration. In certain preferred embodiments, the droplet size of the delivered formulations further provides for an increase in surface area by being sprayed sublingually as opposed to being placed under the tongue, e.g., with a dropper. The size of the spray particles and shape of the spray pattern also may contribute to whether the active ingredient is absorbed into body systems other than the oral mucosa (e.g., lungs).

The spray pump device may be premetered or, alternatively, the device may be device-metered. Premetered devices preferably contain previously measured doses or a dose fraction in some type of units (e.g., single unit dose amount of solution, single or multiple blisters or other cavities) that may be included in the device during manufacture or by the subject before use. Typical device-metered units have a reservoir containing formulation sufficient for multiple doses that are delivered as metered sprays by the device itself when activated by the subject. The device may be metered both in the amount of drug substance delivered (i.e., the dosage per actuation), as well as the length of time between each dosage. Limiting the time between each dosage can prevent over-use by limiting how often a dosage can be delivered to the subject.

A typical spray delivery device includes a base unit, a discharge actuator, an orifice for the formulation to be released from the device, and a reservoir. Preferably the reservoir is filled with the drug substance and other excipients (e.g., liquid vehicle, flavors, sweeteners, etc. as discussed here above) prior to dispensing to the subject, e.g., at the manufacturing site. The reservoir preferably defines a measured amount of the peptide as described here above to be discharged upon activation. The reservoir body may be any acceptable material, for example, formed simply by a section of a cylindrical hollow of a plastic, steel, such as stainless steel, transparent material, or the like so that its production is very simple. An actuator, which is movable relative to the orifice for activating discharge, may be provided on or with the device. In the course of the actuating movement, the reservoir opens, e.g. by puncturing, to administer a single dosage through an orifice. During a part of the actuating travel following the starting position an elevated pressure is built up. In a subsequent portion of the actuating movement continuing in the same direction, the medium may be relieved of the pressure at one of the sides and communicated to an orifice. In such a manner, the medium is pushed from the reservoir and through the orifice by the action of pressure.

Typically as the liquid formulation leaves the orifice, the liquid droplets follow a trajectory which is influenced by the orifice shape, as well as by pressure asserted. In some embodiments, the droplet size, spray geometry, and the spray pattern are dependent on the design of the pump and/or the properties of the formulation. In certain embodiments, the orientation of the actuator, pump design, and the properties of the formulation will influence the spray symmetry and the shape. The spray pattern may also be optimized to disperse the droplets over a wider pathway thereby increasing the surface area through which the compound can be absorbed and reducing the swallowing reflex. The spray device may further be designed to facilitate ease of subject use and placement of the administered spray to specific regions of the oral mucosa.

The previous preferred spray embodiment is not intended as limiting. In practicing the invention, formulations containing the peptide as described here above may alternatively or additionally be provided as other sublingual and/or buccal compatible dosage forms. For example, sublingual liquids provided as liquids compatible with administration by a dropper or similar device are contemplated by the Applicant. In another example, the sublingual formulation can be packaged in pharmaceutically acceptable unit dose ampoules with snap-off tops to permit the opened ampoule to be tipped under a subject's tongue to dispense a single dose of the formulation.

One object of the invention is a method for inducing or facilitating analgesia or for preventing or treating pain in a subject in need thereof, comprising the administration of a therapeutically effective amount of the peptide as described here above or the pharmaceutical composition as described here above via a transbuccal route.

One object of the invention is the peptide as described here above or the pharmaceutical composition as described here above for use in inducing or facilitating analgesia for preventing or treating pain in a subject in need thereof, wherein said peptide or pharmaceutical composition is administered via a transbuccal route.

In one embodiment of the invention, the pain to be prevented or treated is an inflammatory pain or an acute or chronic pain, such as for example post-operative pain, post-traumatic pain, or pain associated with cancer.

In one embodiment of the invention, the subject is a mammal, preferably a human. In another embodiment, the subject may be a human companion animal or agricultural livestock.

In one embodiment of the invention, the peptide or the pharmaceutical composition as described here above is administered via a sublingual route.

In another embodiment of the invention, the peptide or the pharmaceutical composition as described here above is administered via a buccal spray or a sublingual spray.

In another embodiment of the invention, the peptide or the pharmaceutical composition as described here above is administered via a lollipop.

In another embodiment of the invention, the peptide or the pharmaceutical composition as described here above is administered via a film.

In another embodiment of the invention, the peptide or the pharmaceutical composition as described here above is administered via a disintegrating tablet.

In another embodiment of the invention, the peptide or the pharmaceutical composition as described here above is administered via a chewing-gum.

According to the invention, the therapeutic dosage of the peptide will vary according to, for example, the particular use for which the treatment is made, the health and condition of the subject, the severity of the pain, the cause of the pain, the age of the subject, and the judgment of the prescribing physician. Effective amounts of the peptide contemplated by the present invention will vary depending on the severity of the pain and the health and age of the recipient. In general terms, effective amounts may vary from 0.01 ng/kg body weight to about 100 mg/kg body weight. Alternative amounts include from about 0.1 ng/kg body weight to about 100 mg/kg body weight or from 1.0 ng/kg body weight to about 80 mg/kg body weight.

Another object of the invention is a method for treating or preventing pain comprising the steps of applying to the oral mucosa of a subject the peptide or pharmaceutical composition as described here above, wherein said peptide is absorbed through said oral mucosa and produces analgesia.

Another object of the invention is a method for treating or preventing pain comprising administering to the oral mucosa of a subject the pharmaceutical composition as described here above, in an amount effective to treat or prevent pain in said subject upon administration, wherein said pharmaceutical composition provides a physiologically active amount of the peptide into the systemic circulatory system of said subject at a rate that produces an analgesic effect.

In one embodiment of the invention, said pharmaceutical composition is adapted for transmucosal administration to a subject by applying said pharmaceutical composition to a mucous membrane of the oral cavity of said subject.

In another embodiment of the invention, said pharmaceutical composition is adapted for sublingual administration to a subject by applying said pharmaceutical composition to a mucous membrane under the tongue of said subject.

In another embodiment of the invention, the method for preventing or treating pain comprises spraying the oral mucosa of the subject with a metered dosage of a spray composition comprising the peptide as described here above in an effective amount to provide transmucosal absorption of a pharmaceutically effective amount of the peptide through the oral mucosa into the systemic circulatory system of the mammal.

According to the invention, the analgesia or the treatment of pain is effective from to 2 min, to 1h, 2h, 3h, 4h, 5h, 6h, 8h, 12h, 16h, 20h, 24h, 36h, 48h post administration.

In one embodiment of the invention, the pain treated or prevented by the pharmaceutical composition of the invention is a symptom of a condition associated with nitric oxide synthase.

Examples of such conditions include: headache (e.g., migraine headache (with or without aura), chronic tension type headache (CTTH), migraine with allodynia, medication overuse headache, cluster headache, chronic headache, or transformed migraine); neuropathic pain (AIDS associated painful neuropathy, central post-stroke pain (CPSP), diabetic neuropathy, chemotherapy induced neuropathic pain (e.g., paclitaxel, cis-Platin, Doxorubicin etc.), postherpetic neuralgia, or trigeminal neuralgia), chronic inflammatory pain (e.g., pain that results from osteoarthritis, rheumatoid arthritis, ankylosing spondylitis, psoriatic arthritis, undifferentiated spondyloarthropathy, or reactive arthritis); visceral pain; neuroinflammation; medication-induced hyperalgesia and/or allodynia (e.g., opioid- induced hyperalgesia/allodynia or triptan (5-HTID/IB agonists)-induced hyperalgesia or allodynia); acute pain (optionally in combination with an opioid treatment); chronic pain; bone cancer pain; chemical dependencies or addictions (e.g., drug addiction; cocaine addiction; nicotine addiction; metamphetamine-induced neurotoxicity; ethanol tolerance, dependence, or withdrawal; or morphine/opioid induced tolerance, dependence, hyperalgesia, or withdrawal); CNS disorders (e.g., epilepsy, anxiety, depression (alone or in combination), attention deficit hyperactivity disorder (ADHD), psychosis, or dementia); neurodegenerative diseases or nerve injury (e.g., acute spinal cord injury, AIDS associated dementia, Parkinson's disease, Alzheimer's disease, amyotrophic lateral sclerosis (ALS), Huntington's disease, multiple sclerosis, neurotoxicity, or head trauma); cardiovascular related conditions (e.g., stroke, coronary artery bypass graft (CABG) associated neurological damage, hypothermic cardiac arrest (HCA), post-stroke pain, cardiogenic shock, reperfusion injury, or vascular dementia); or gastrointestinal disorders (e.g., ileostomy-associated diarrhea or dumping syndrome). In another embodiment, the pharmaceutical composition of the invention can be used for the treatment of post-operative pain, post-traumatic pain, or pain associated with cancer.

In another embodiment, the pharmaceutical composition of the invention can be used for the treatment of chronic pain with central sensitization. In another embodiment, the pain with components of central sensitization is neuropathic pain. In another embodiment, the neuropathic pain is selected from postherpetic neuralgia, diabetic neuropathy, central (thalamic) pain, post-stroke pain, HIV-associated pain, phantom limb pain, neuropathic pain resulting from post-surgical trauma or nerve injury, and chemotherapy-induced neuropathies.

In another embodiment, the pharmaceutical composition of the invention can be used for the treatment of chronic inflammatory pain. In other embodiments, the chronic inflammatory pain relates to ankylosing spondylitis, reactive arthritis (Reiter's syndrome), psoriatic arthritis, undifferentiated spondyloarthropathy, rheumatoid arthritis, and osteoarthritis.

In another embodiment, the pharmaceutical composition of the invention can be used for the treatment of headaches with underlying mechanisms of central sensitization. In other embodiments, headache is selected from migraine, chronic tension type headache (CTTH), cluster headache, transformed migraine, and medication overuse headache. Still other embodiments include use of the pharmaceutical composition of the invention for treatment of interstitial cystitis or opiate withdrawal or migraine prophylaxis.

Advantages of transbuccal administration of the peptide or pharmaceutical composition of the invention:
- Bioavailability of the peptide by transbuccal delivery is independent of observed variations in bioavailability of drugs from the gut secondary to GI abnormalities,
- Bioavailability of the peptide by transbuccal delivery is independent of variations in bioavailability secondary to pH changes,
- Bioavailability of the peptide by transbuccal delivery is more convenient for patients with swallowing difficulties,
- It allows self-administration by the subject,
- It provides a rapidly acting, potent non-narcotic analgesic that ameliorates, manages, cures, prevents or treats pain,
- It allows a diminution in the dosage of peptide used, thereby reducing the likelihood of deleterious side effects and providing a cost benefit for the manufacturer,
- It avoids tolerance to the peptide,
- It does not induce an antibody response,
- It does not induce respiratory depression and has no effect on heart rate.

### BRIEF DESCRIPTION OF THE FIGURES

**Figure 1****:** Time course of the antinociceptive effect on the hot plate after sublingual administration of the peptide (0.4 mg/kg, 0.5 ml/kg). N=10. *p<0.05 compared to control.
**Figure 2****:** Log dose-response curve of the peptide after sublingual administration (0.3-30 mg/kg, 0.5 ml/kg at 3h post-dose). N=5-10 mice.
**Figure 3****:** (A) Effect of the peptide (1 mg/kg) administrated by sublingual route in the formalin model. N=10. *p<0.05 compared to control. (B) Effect of the peptide on the acute phase of response after formalin injection. N=8 (saline) and 13 (peptide). *p<0.05 compared to control.
**Figure 4****:** Plasma concentration of the peptide (1-25 mg/kg, 0.5 ml/kg) at various times after administration by sublingual route in mice. N=3. The first point was obtained immediately after drug administration which is less than 1 min.
**Figure 5****:** Plasma concentration of the peptide (1-25 mg/kg, 0.5 ml/kg) at various times after administration by sublingual route in mice. N=3.
**Figure 6****:** (A) Plasma concentration of the peptide (25 mg/kg, 0.5 ml/kg) at various times after different routes of administration. N=3 (B) Chart showing bioavailability of the peptide using various routes of administration. Data were calculated using area under curve (AUC) for each route and compared to intravenous route.
**Figure 7****:** Acute effects of high dose morphine and peptide on GI transit distance.
Morphine (M) at 0.5-50 mg/kg exhibits a dose dependent inhibitory effect on the GI transit activity. The peptide (P) at both 5 and 50 mg/kg does not show any significant effect. N= 5 to 8 mice. *p<0.05 significantly different from control by independent sample t-test.
**Figure 8****:** (A) Example of blood pressure charts taken from three different animals with similar readings indicating a dose dependent drop upon point of administration of the peptide via intravenous route. (B) Example of blood pressure charts taken from three different animals with similar readings indicating no change in blood pressure upon administration of the peptide via sublingual and intraperitoneal route.
**Figure 9****:** Effect of the peptide (P) and morphine (M) on motor-coordination in SA mice. N=8-10.
**Figure 10****:** (A) Pre-treatment - locomotive activities of SA mice. N=8. (B) Acute effects of morphine (M) and the peptide (P) on locomotor activities of SA mice. N=8.
**Figure 11****:** Anxiety scores of Peptide and Diazepan in rats in Open-Field Test. N=3. *P<0.01.
**Figure 12****:** Chart indicating average time spent by the rats in open/closed arms in Elevated-plus Maze Test. N=3.

### EXAMPLES

### Materials

The sequence SEQ ID NO: 3 was synthesized by Bachem, Switzerland. All experiments were performed using GLP grade peptide.

### Example 1: the Hot Plate model

The hot plate model was used to evaluate the time and dose dependence of the antinociceptive effect of the peptide after sublingual administration. Different mice were used at each time point or dose. The peptide was dissolved on saline unless specified.

### Methods:

Swiss albino mice (20-25 g, male) were placed on a hot-plate (55°C) and confined by a circular transparent restrainer of 13 cm height and diameter. The response latency was measured from the time the mouse was placed on the hot-plate to the time it exhibited one of the following endpoints: licking of a paw, stomping of a limb, and jumping (defined as a momentary lifting of all 4 limbs off the hot-plate). Mice were tested before and at an appropriate time after drug administration. Statistical analysis was performed by paired t-test between pre-dose and post- dose latencies.

### Results:

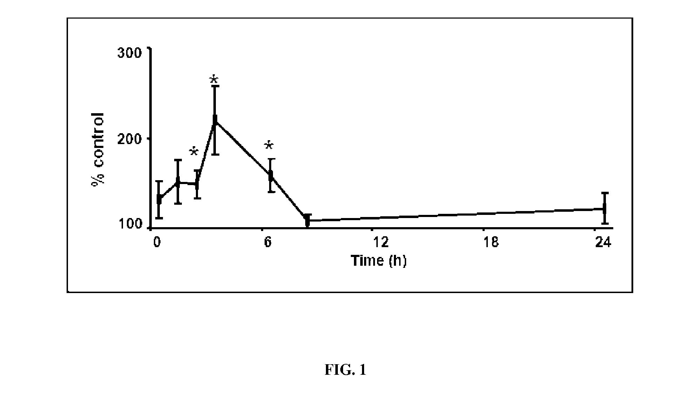

At a dose of 0.4 mg/kg after sublingual administration, the peptide exhibits significant antinociceptive effect for the duration of 1 - 6 h post-dose (Fig. 1). The antinociceptive effect appeared to peak at 3 h.

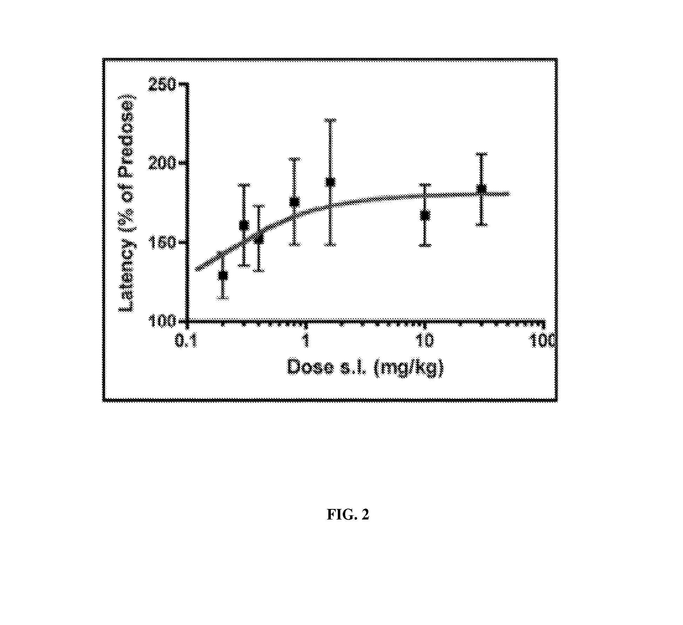

Data presented in Fig. 2 shows the dose-dependency of the antinociceptive effect of the peptide in the hot-plate model (sublingual administration, 3 h post-dose) with an ED50 of 0.18 mg/kg and Emax of 180% of pre-dose latency. In comparison, using morphine (intraperitoneal administration, 30 min post-dose) as a positive control, we obtained an ED50 of 0.82 mg/kg and Emax of 250% of pre-dose latency. (Data were fitted to the equation Y=E₀+(Eₘₐₓ-E₀).D/(D+ED₅₀) where E₀=100.)

In conclusion, the peptide exhibits a time- and dose-dependent antinociceptive activity in the hot-plate model (thermal acute pain) upon sublingual administration.

### Example 2: the Acetic Acid-induced Writhing model

### Methods:

Swiss albino mice (20-25 g, male) were administered with the peptide and at the appropriate post- dose time acetic acid (0.9% solution) was injected intraperitoneally (10 ml/kg). The mice were then placed in a transparent observer box and the total number of writhes per animal in the 15 min period after injection of acetic acid was recorded. A writhe is typically characterized by contractions of the abdominal musculature followed by extension of the hind limbs. Statistical comparison was done by either Student's t-test or one-way ANOVA.

At 1 h post administration time, acetic acid (0.9% solution) was injected intraperitoneally and the total number of writhes per animal was recorded, as described above. Upon completion of the experiment the animals were sacrificed and skull exposed. The accuracy of the injection was confirmed via injection of methyl-blue dye into the injection aperture left by the original injection. Only results from mice showing coloration of the ventricles are accepted.

### Results

The results in Table 1 show that the peptide has an antinociceptive effect when administrated via a sublingual route (1 mg/kg).

**Table 1: Effect of morphine (1 mg/kg, intraperitoneal) and the peptide (1 mg/kg, sublingual) in the writhing model.**

| | % saline control | |
|---|---|---|
| | Morphine (n=8) | The peptide (n=6) |
| Time to first writhe | 190.75* | 168.93* |
| Writhes in first 5 min | 12.5* | 63.95 |
| Writhes in subsequent 10 min | 48.56* | 69.07* |
| Total writhes in 15 min | 38.18* | 67.49* |

### Example 3: the Formalin model

### Methods

Sprague-Dawley rats (about 200 g, male) were sublingually administered with the peptide and at 1 h post-dose time a formalin solution (2.5%, 100 µl) was injected subcutaneously in the dorsal aspect of the right hind paws. Rats were then placed in a transparent observation box. Observations were made real time for 1 h by an observer. Simultaneously, video recording was also made with a camera mounted underneath the box. The number of flinches of the formalin- injected limb was recorded every minute for the first 10 min (the acute phase). Thereafter, flinches were recorded for the first min of every 5 min for the next 50 min (the delayed phase). The video recording was viewed later for confirmation of results. Statistical comparison was done by either Student's t-test or one-way ANOVA.

### Results:

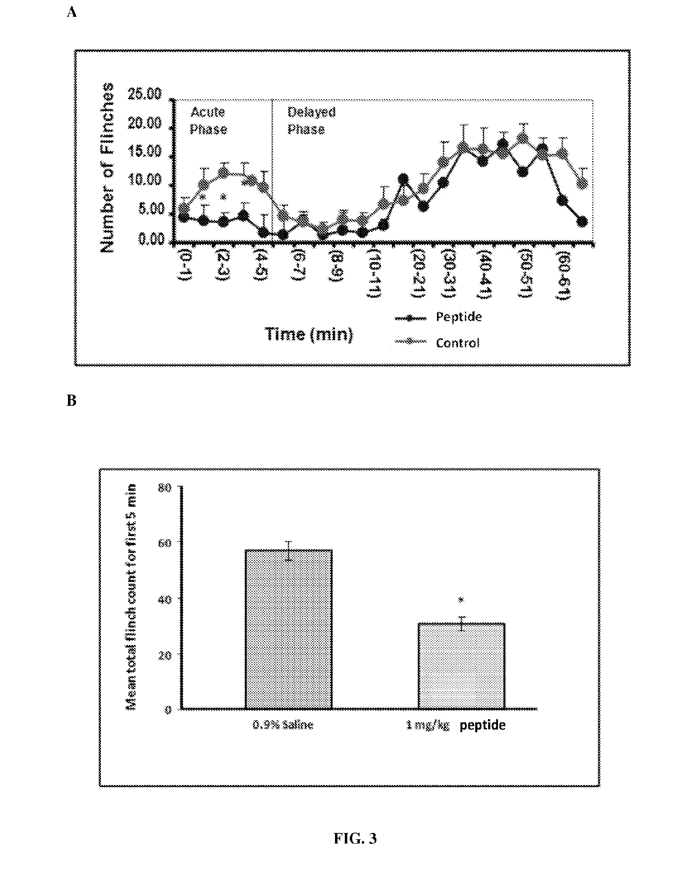

Fig. 3A shows that the peptide (1 mg/kg) significantly reduced the number of flinches of the formalin-injected hind limb compared to saline-treated controls in the acute phase (first 5 min after formalin injection). However, no significant effects were observed in the delayed phase. In a separate experiment only the number of flinches in the first 5 min was recorded and the peptide showed antinociceptive effects (Fig. 3B).

### Conclusions:

The peptide upon sublingual administration exhibits antinociceptive activity in the formalin-induced acute phase pain, but not the delayed phase pain.

### Example 4: Pharmacokinetics

### Methods:

The peptide (1, 5, 15 and 25 mg/kg, 0.5 ml/kg) was administered sublingually to mice. Upon reaching specified time points of 1, 5, 15, 30, 60 and 120 min post-dose, each mouse was sacrificed and blood samples were collected via cardiac puncture. Plasma samples were then extracted and analyzed under LC/MS/MS. Systemic exposure of the peptide was also studied through intravenous, oral (gavage) and intraperitoneal routes.

### Results:

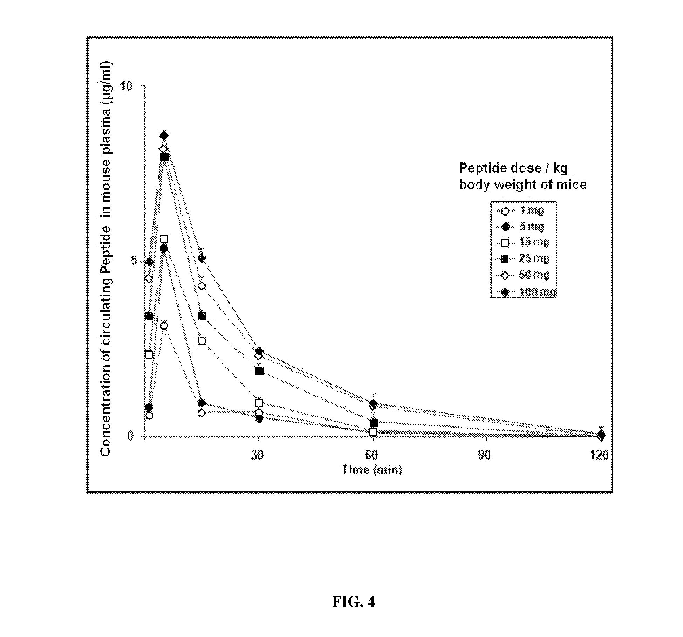

Fig. 4 shows that the peptide was detectable rapidly after sublingual administration confirming systemic exposure. Plasma concentrations increased with increasing doses. At doses below 5 mg/kg, plasma concentration of the peptide was negligible by 30 min after administration.

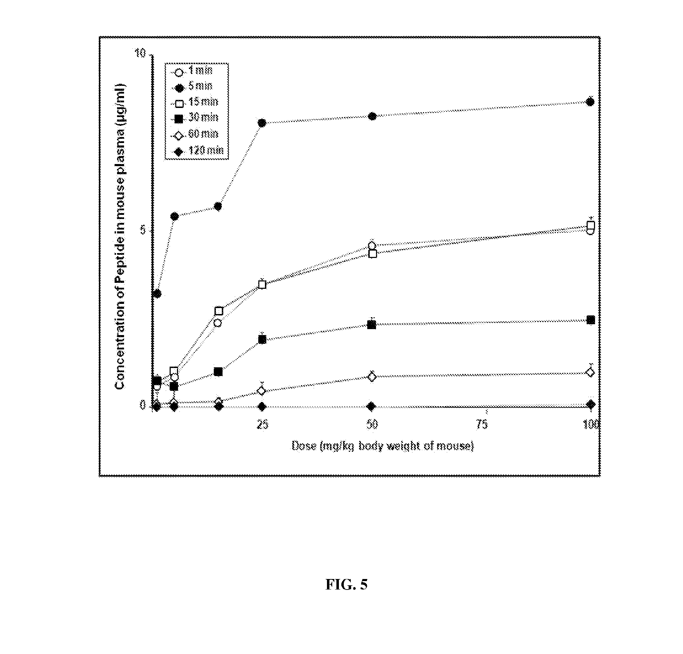

The peptide reached highest level in the plasma 5 min after the sublingual administration. At this time point, the absorption and removal probably reaches the equilibrium at higher amounts of the peptide (Fig. 5).

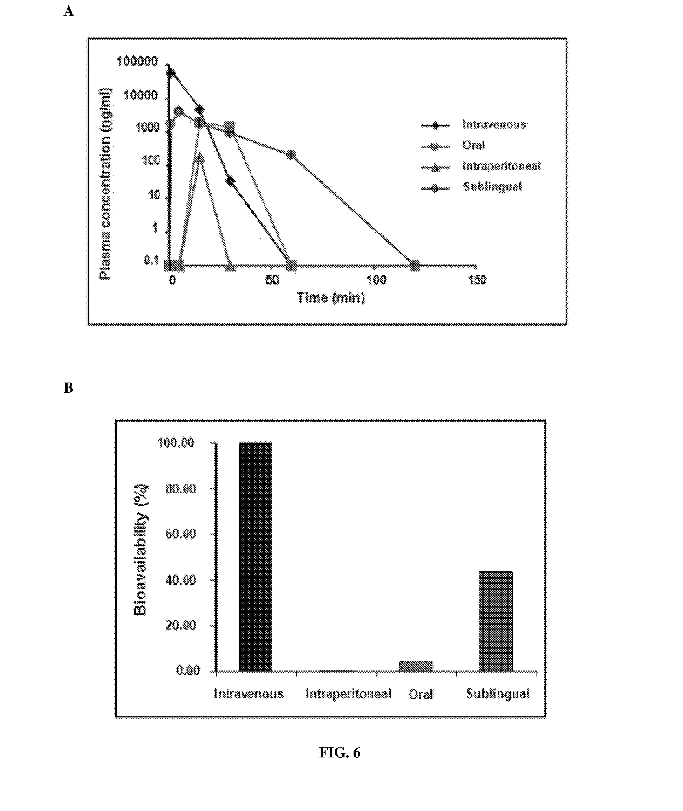

Fig. 6 shows that the peptide was detectable rapidly after intravenous, sublingual, oral and intraperitoneal administration.

### Conclusion:

These results demonstrated that the peptide was effectively absorbed when administered by the sublingual, oral, and intraperitoneal routes. The amount of peptide detectable in the plasma disappeared very rapidly within minutes. This contrasted with the observed long duration of analgesic effect up to 6 h after sublingual administration (Fig. 1). The analgesic effect therefore did not require the continuous presence of peptide in the plasma. Such behavior of PK-PD is common with a number of peptide drugs.

From Fig. 6B, bioavailability of the peptide administered via sublingual route was found to be the highest at 44% compared to oral (4%) and intraperitoneal (0.5%) route

As a comparative, synthetic Oxytocin (Pitocin / Syntocinon: 1007.19 molecular weight), an approved therapeutic peptide that is used to help start or continue labor and to control bleeding after delivery has a similar molecular weight as the peptide. Various studies have been conducted to investigate the administration of Oxytocin via sublingual route. The bioavailability by the sublingual route of Oxytocin prepared in physiological saline solution has been reported to be 0.007 - 0.07%. In a separate study, bioavailability of oxytocin was enhanced to 27% with a formulation change to include 0.75% w/w sodium taurocholate (tablet form), which is lower than the peptide (44%).

### Example 5: Toxicity

### 5.1. Single Dose Regimen

### Methods:

Swiss albino mice were administered with the peptide at 8 mg/kg or 80 mg/kg (intraperitoneal) on day 1 and then observed for 14 days. Control mice received saline.

At the end of the period, all mice were sacrificed, dissected and observed for any obvious abnormality. Weights of the major organs, including brain, heart, lung, liver, spleen and kidney, were recorded.

### Results:

A single high dose of Peptide up to 80 mg/kg did not cause any obvious toxicity in mice (Table 2). There was no significant difference in body weights among the group on both day 1 and 14. All organs appeared normal and no differences in weight were observed among the 3 groups (data not shown).

**Table 2: Effects on body weight and mortality after a single dose of the peptide over a 14-days period**

| | **Control** | **8 mg/kg, intraperitoneal** | **80 mg/kg, intraperitoneal** |
|---|---|---|---|
| **Body Weight (g)** | | | |
| **Day 1** | 25.7 ±0.9 | 26.2 ±0.4 | 25.8 ±0.6 |
| **Day 14** | 33.0 ±0.6 | 31.7 ±0.5 | 31.1 ±0.7 |
| **Appearance** | normal | normal | normal |
| **Death** | nil | nil | Nil |
| **N** | 6 | 12 | 12 |

### 5.2. Multiple Dose Regimen

### Methods:

Swiss albino mice were administered with the peptide at 8 mg/kg or 80 mg/kg (intraperitoneal) once daily for 14 days. Control mice received saline. At the end of the period, all mice were killed, dissected and observed for any obvious abnormality. Weights of the major organs, including brain, heart, lung, liver, spleen and kidney, were recorded.

### Results:

Multiple high doses of peptide up to 80 mg/kg for 14 days did not cause any obvious toxicity in mice. There was no significant difference in body weights among the group on both day 1 and 14 (Table 3). All organs appeared normal and no differences in weight were observed among the 3 groups (data not shown). Only one remarkable event during this experiment was the death of one mouse in the 8 mg/kg group on day 12. Since it occurred in the 8 mg/kg group and not the 80 mg/kg group, it was concluded that this death was probably unrelated to the peptide treatment.

**Table 3: Effects on body weight and mortality after multiple doses of the peptide over a 14-days period**

| | **Control** | **8 mg/kg, Intraperitoneal, daily** | **80 mg/kg, Intraperitoneal, daily** |
|---|---|---|---|
| **Body Weight (g)** | | | |
| **Day 1** | 21.3 ±0.5 | 21.4 ±0.8 | 21.3 ±0.5 |
| **Day 14** | 30.0 ±0.7 | 30.0 ±0.4 | 28.1 ±1.2 |
| **Appearance** | normal | normal | normal |
| **Death** | nil | nil | Nil |
| **N** | 12 | 12 | 12 |

The multiple dose toxicity testing was repeated exactly as described above except that C57 mice were used instead of Swiss albino mice. Similar results were obtained in terms of body weights, vital organ weights and behavior of control and treated animals (data not shown). No death occurred in any groups.

### Conclusion:

The peptide exhibits very low toxicity even at high concentrations of up to 80 mg/kg (intraperitoneal administration) (200 x effective dose) multiple dosing up to 14 days. No mortality was observed for this dosing plan using two strains of mice.

### Example 6: Acute High Dose Toxicity

### Methods:

Acute toxicity was determined by both oral and intravenous administration of the peptide. Three mice were given a dose of peptide at 2 g/kg orally by gavage, 5 ml/kg. In a separate experiment, mice were given a dose of peptide at 125 mg/kg or 62.5 mg/kg intravenously via a tail-vein, 5 ml/kg.

### Results:

Within minutes after oral administration (2 g/kg, 5000 x effective dose), mice appeared sedated with much decreased activity but recovered after about 1 h. They appeared completely normal thereafter up to 2 weeks.

After intravenous administration of the peptide (125 mg/kg), four out of nine mice exhibited jerky movements similar to pro-convulsion behavior but actual convulsion was not observed. These four mice died within 5-10 min. The other five appeared heavily sedated for several hours. They gradually recovered and appeared quite normal the next morning. Another three mice were given a dose of peptide at 62.5 mg/kg intravenously. These three mice also appeared sedated after peptide but recovered after several hours. It is thus concluded that the LD50 of peptide administered intravenously is about 125 mg/kg. The surviving mice appeared completely normal thereafter up to 2 weeks.

Table 4 shows the body weights of these mice before peptide and 2 weeks later compared to controls. For the 125 mg/kg intravenous group, only the 5 mice that survived were included.

**Table 4: Acute toxicity of the peptide after high doses administered intravenously or orally**

| | **Control** | **2g/kg, oral** | **62.5 mg/kg, intravenous** | **125 mg/kg, intravenous** |
|---|---|---|---|---|
| **Body Weight (g, predose)** | 30.7±1.9 | 29.3±1.5 | 30.0±1.0 | 28.6±1.3 |
| **Body Weight (g, 2 weeks postdose)** | 35.3±2.2 | 33.0±1.7 | 34.7±1.5 | 33.6±1.5 |

### Conclusion:

The LD50 of the peptide administered intravenously was determined to be about 125 mg/kg in mice. However, mice administered up to ∼20 times higher dose of 2 g/kg orally appeared heavily sedated following administration but recovered within 1 h and remained alive throughout the entire duration of the study of 2 weeks.

### Example 7: Investigations on potential adverse effects

### 7.1. Effects of the peptide on Gastrointestinal System (GI motility)

### Methods:

Swiss albino mice (30-35 g) were injected with saline, the peptide (5 mg/kg, 50 mg/kg intraperitoneal) or morphine (0.5 mg/kg, 5 mg/kg, 50 mg/kg intraperitoneal). The experimenters are blind to the identity of the solutions. All mice were administered 5 mg charcoal (0.1 ml of 50 mg/ml charcoal solution) via oral gavage immediately after drug administration. At 30 min post- drug / charcoal administration, the mice were sacrificed by cervical dislocation and the GI tract was exposed and the location of the charcoal identified. The distance the charcoal traveled in the intestine from the stomach was measured and expressed as % of total length of the GI tract.

### Results:

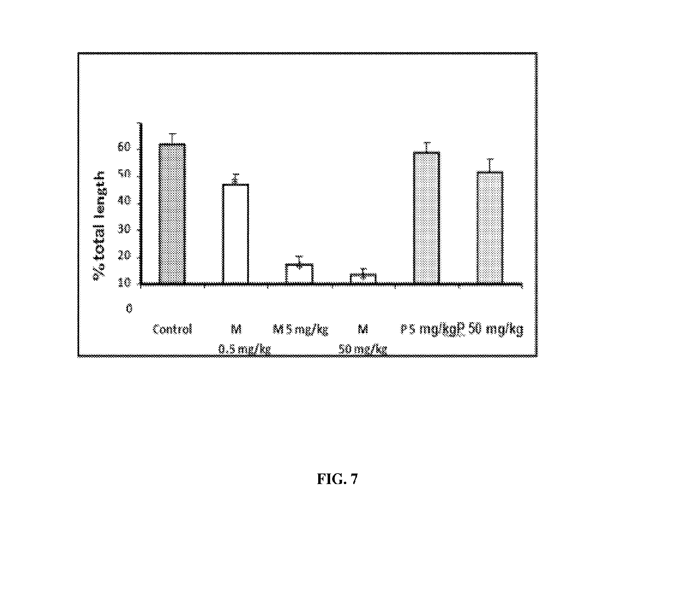

Morphine at 0.5 to 50 mg/kg had dose-dependently decreased GI motility as shown by the marked reduction in the distance traveled by the charcoal in the intestine. In contrast, the peptide at 5 mg/kg and 50 mg/kg did not show any significant reduction in GI transit distance (Fig. 7).

In conclusion, the peptide even at a high concentration of 50 mg/kg did not cause any significant reduction of GI transit distance. Thus, the peptide has no adverse effects on GI motility that is common to opioids analgesics.

### 7.2. Effects of the peptide on Cardiovascular System

### Acute Effect of Intravenous Peptide on Blood Pressure

### Methods:

Sprague-Dawley rats (∼300 g) were anesthetized and the carotid artery was cannulated for blood pressure measurement. Drugs were administered by the pre-determined route. A pulse transducer was secured across the thoracic region of the rat to allow measurement of respiration rate. Blood pressure was recorded for at least 15 min or until a relatively stable blood pressure was observed.

### Results:

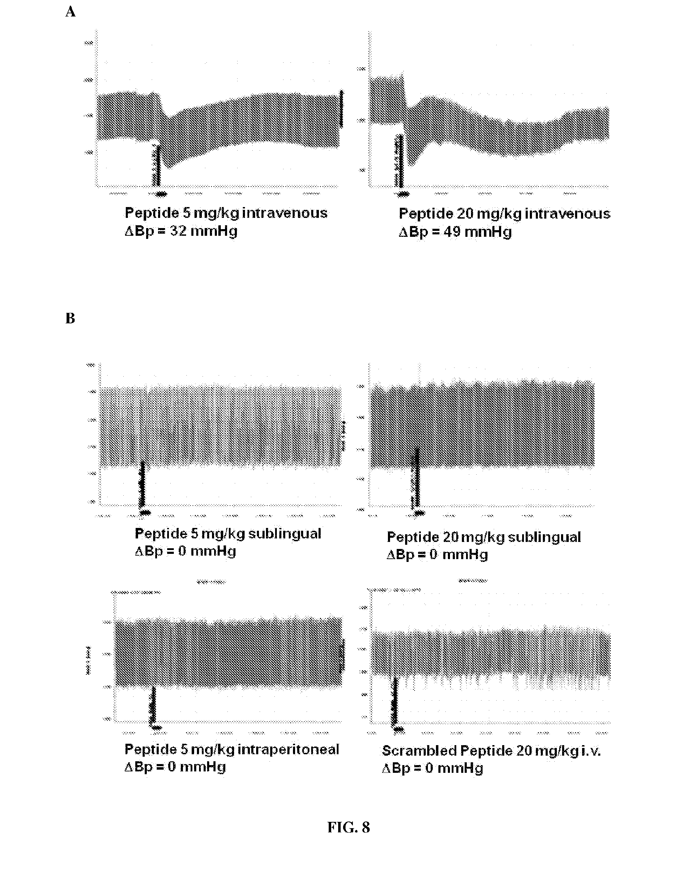

Peptide at both 5 and 20 mg/kg decreased in blood pressure when administered by the intravenous route. The magnitude of effects varies markedly from rat to rat, the tracings (Fig. 8A) show two examples of marked responses obtained.

In contrast, the same doses of peptide (5 and 20 mg/kg) administered by the sublingual or intraperitoneal routes did not cause any changes in blood pressure (Fig. 8B). To ascertain that the blood pressure effect observed after intravenous administration was indeed caused by the peptide, the scrambled peptide was administered in the same manner and found not to cause any effect on blood pressure.

No significant changes in heart rate and respiration rate were observed in all experiments.

In conclusion, the peptide at 5 and 20 mg/kg exhibits transient blood pressure lowering effects only when administered intravenously. This effect is not observed with sublingual or intraperitoneal administration.

### 7.3. Effects of the peptide on Central Nervous System

### Rotarod Test

### Methods:

Swiss albino mice (∼20-30 g) were first trained on the rotarod, which accelerated from 0-20 rpm over 5 min, prior to the experiment. Only mice that were capable of staying on the rotarod for the entire 5 min period were used in this experiment. Mice were either administered with the peptide (1 or 10 mg/kg, sublingual), or morphine (20 mg/kg, subcutaneous). After drug administration, the animals are placed on the rotarod and the time taken for the mice to slip was recorded (up to 5 min), and subsequently at 1h, 2h and 3h post peptide, and at 30 min, 1h30 min and 2h30 min post morphine. Statistical comparison was done by either Student's t-test or one-way ANOVA.

### Results:

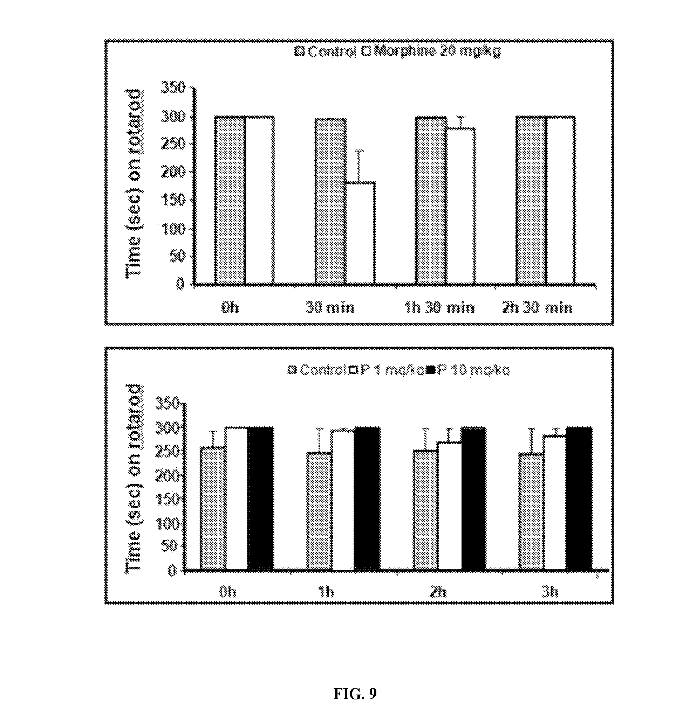

No statistical significant effects (figure 9) were observed for both morphine and peptide treated groups although a near significant decrease in performance can be observed for the morphine treated group at 30 min post drug administration. (p=0.056, t test)

In conclusion, no significant neurologic deficits were observed upon sublingual administration of the peptide for up to 3 h.

### Spontaneous Locomotor Activity in the Mice.

### Methods:

Swiss albino mice (∼30 - 40 g) were first placed into their respective chambers with a 20 min pre-treatment recording. Only mice showing at least 50 counts / 5 min are used in the experiments. Mice were administered saline (control), peptide (5 or 50 mg/kg) or morphine (50 mg/kg). Experimenters were blind to the identity of the drug solutions. After drug administration, the animals were placed into their individual chambers and their spontaneous locomotor activity was recorded by video tracking and the data were recorded for a period of 4 h. Statistical comparison was done by 2-way ANOVA.

### Results:

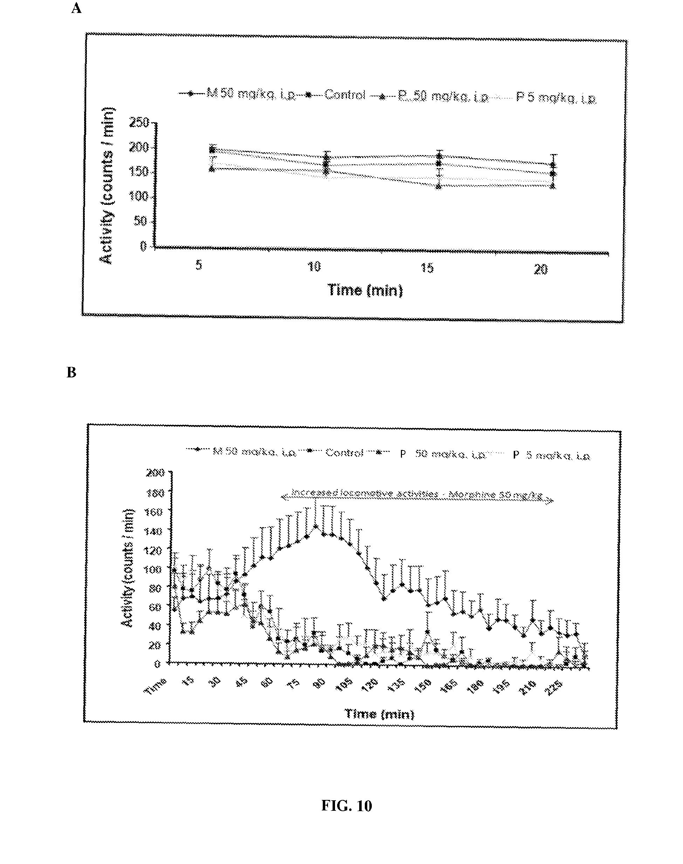

As shown on Fig. 10A, no significant difference in activity was observed at any time points for a 20 min period pre-drug administration.

In conclusion, the peptide does not cause any significant sedation or other neurological deficits up to 3 h post dose.

Morphine (50 mg/kg, intraperitoneal) showed a significant increase in locomotive activities between 60 min and 220 min post drug administration (n = 8, one-way ANOVA, p<0.05). In contrast, no significant difference was shown from either the peptide-treated groups when compared to control within 4 h of drug administration. Comparing among groups, significant difference was shown for the morphine treated group as well (two-way ANOVA, F = 7.205, 0.001) (Fig. 10B).

In conclusion, behavioral excitation, restlessness and increased locomotor activity, was observed in the Morphine-treated mice. No such effect was evident in the peptide-administered mice.

### Open-Field Test in rats

The open field test is generally used to assess anxiety level of rodents due to their natural aversion of open space and thus their preference to stay near the side rather than venturing to the more open central part of the open field.

### Methods:

The open field was divided into 16 equal size squares (25 x 25 cm). The 4 squares at the centre are assigned a low anxiety score (0) while those at the 4 corners are assigned a high anxiety score (2). The remaining 8 squares are assigned a medium score (1). A rat entering each square will be given the score assigned to that square. The sum total obtained within the 5-min test period divided by the number of squares entered is the anxiety score for that rat. Diazepam was used as positive control. Statistical comparison was done by Student's t-test.

### Results:

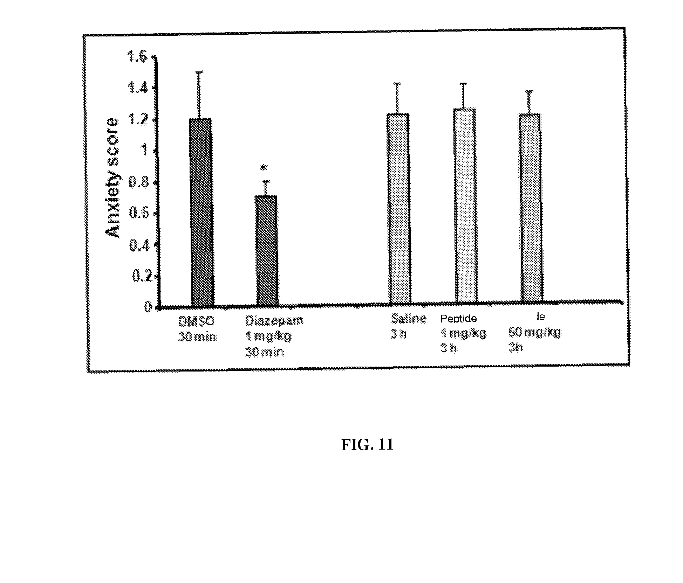

The peptide given intraperitoneally (1 or 50 mg/kg) did not show any effect on the anxiety scores obtained when compared to the saline control. Therefore, the peptide neither increased nor decreased anxiety levels. In contrast, a well-studied anti-anxiety agent diazepam decreased the anxiety score significantly [t (14) = 3.048, P < 0.01] (Fig. 11).

### Elevated-Plus Maze Test

### Methods:

The Elevated-Plus Maze Test is generally used to assess anxiety level of rodents due to their natural aversion for height. Two opposite arms of the elevated-plus maze are enclosed by high walls (closed arms) while the other two are not (open arms). The test animals (rats) naturally prefer and spend more time in the closed arms.

### Results:

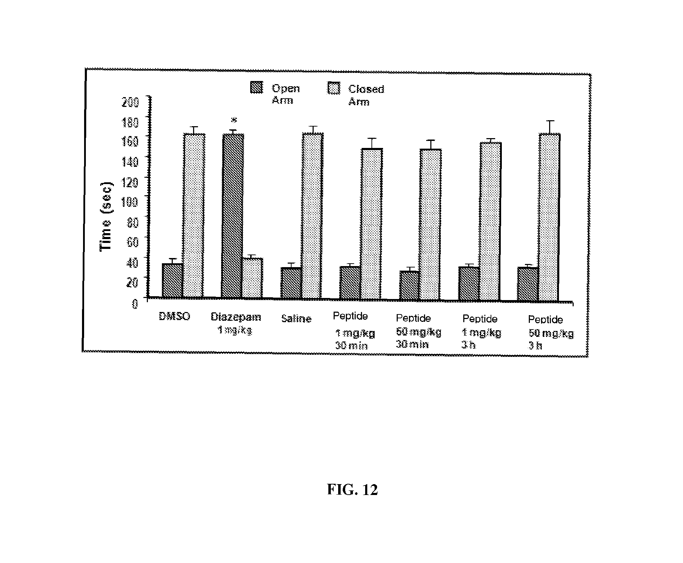

As shown in Fig. 12 statistical analysis showed that, as compared to saline, the peptide (1 or 50 mg/kg, 30 min or 3 h post-dose) did not produce any significant changes in time spent in the open or closed arms. In contrast, diazepam reversed the times spent in open and closed arms when compared to DMSO-controls.

In conclusion, in the open field and elevated-plus maze test for anxiety levels for rats, the peptide did not alter the natural anxiety levels of rats when compared to the known anti-anxiety drug, diazepam.

## Claims

1. A pharmaceutical composition comprising a peptide of 5 to 50 amino acids which comprises the amino acid sequence NPFPTX₁X₂KRX₃X₄ (SEQ ID NO: 2) wherein X₁, X₂, X₃, and X₄ may be the same or different and each is an amino acid residue, wherein said composition is in a form suitable for a transbuccal administration.

2. The pharmaceutical composition according to claim **1,** wherein the peptide comprises the acid sequence NPFPTX₁X₂KRX₃X₄ (SEQ ID NO: 2) wherein X₁ is absent or is W, X₂ is absent or is R, X₃ is P or K and X₄ is absent or is G or H.

3. The pharmaceutical composition according to claim **1** or **2**, wherein the peptide comprises the amino acid sequence NPFPTWRKRPG (SEQ ID NO: 3), NPFPTRKRP (SEQ ID NO: 4), NPFPTWRKRP (SEQ ID NO: 5), NPFPTWKRKH (SEQ ID NO: 6) or a derivative, homologue, analogue or mimetic of SEQ ID NO: 3-6.

4. The pharmaceutical composition according to any one of claims **1** to **3**, wherein the composition is in a form suitable for transbuccal administration selected among liquid solutions, orally disintegrating tablets, bioadhesive tablets, microspheres or nanospheres of any shape, film, lollipop, chewing-gum, mouthwash, toothpaste, buccal drops and spray.

5. The pharmaceutical composition according to claim **4**, wherein the composition is in a form of a spray.

6. The pharmaceutical composition according to claim **4**, wherein the composition is in a form of an orally disintegrating tablet.

7. The pharmaceutical composition according to any one of claims **1** to **6**, for use in inducing or facilitating analgesia in a subject in need thereof, wherein said peptide or pharmaceutical composition is administered via a transbuccal route.

8. The pharmaceutical composition according to any one of claims **1** to **6**, for use in preventing or treating pain in a subject in need thereof, wherein said peptide or pharmaceutical composition is administered via a transbuccal route.

9. The pharmaceutical composition according to claim **7** or **8**, wherein the pharmaceutical composition is administered via a sublingual route.

10. The pharmaceutical composition according to anyone of claims **7** to **9**, wherein the pain is an inflammatory pain or an acute or chronic pain.

11. The pharmaceutical composition according to anyone of claims **7** to **10**, wherein the pain is a symptom of a condition associated with nitric oxide synthase.
